# EUROPEAN PATENT SPECIFICATION

(11) **EP 0 483 566 B1**
(45) Date of publication and mention of the grant of the patent: **23.08.1995**
(21) Application number: 91117329.2
(22) Date of filing: 10.10.1991
(51) Int. Cl.: A61B 5/20, A61B 10/00, G01F 23/20

(54) **Automatic quantity of urine-measuring apparatus**
Vorrichtung zur automatischen Urinmengenmessung
Appareil de mesure automatique d'une quantité d'urine

(30) Priority: 27.10.1990 JP 290506/90
(43) Date of publication of application: 06.05.1992
(73) Proprietor: STEC INC., Kyoto (JP); Minami, Yoshiteru, Nerima-ku, Tokyo (JP)
(72) Inventor: Minami, Yoshiteru, Nerima-ku, Tokyo (JP); Oya, Kazuhiro, Kunitachi-city, Tokyo (JP)
(74) Representative: Urner, Peter, Dipl.-Phys.

(56) References cited:
- EP-A- 0 052 495
- EP-A- 0 308 080
- EP-A- 0 393 784
- DE-A- 3 541 649

## Description

### BACKGROUND OF THE INVENTION

### Field of the Invention

The present invention relates to a novel useful automatic quantity of urine-measuring apparatus.

### Description of the Prior Art

In general, the so-called collection of urine, in which the urine is collected in a container, such as urinal, in order to know a quantity of urine discharged from a patient for one day, has been conducted in the hospital.

However, the following problems have occurred in the above described collection of urine. That is to say, a large space for keeping containers, such as urinals, with a name of a person, who discharged a urine, such as patient, marked thereon is required in a lavatory and the like. In addition, since said containers are not single but large in number, a bad smell is given out around a lavatory not only to give a severe uncomfortable feelings to general persons and the like but also to be unsanitary when the containers are kept in a lavatory. Furthermore, even though a person in charge, such as nurse, in the hospital measures a quantity of urine within the respective containers once a day, a problem has occurred in that considerable time and labor are required.

A volumetric sampling device for measuring a whole day urinary output is known from EP-A-0 052 495. An impeller type flow meter for automatically measuring the quantity of a single disposal of urine is known from DE-A-3 541 649.

### SUMMARY OF THE INVENTION

The present invention has been achieved paying attention to the above described matters and it is an object of the present invention to provide a novel useful automatic quantity of urine-measuring apparatus capable of automatically measuring the quantity of urine discharged from a specified person such as patient without requiring a wide space, giving out uncomfortable feelings such as bad smell, and giving bitter feelings to a person in charge.

In order to achieve the above described object, an apparatus for automatically measuring the quantity of urine according to the present invention is characterized in comprising a urinal, a quantity measuring portion provided in a drain outlet of said urinal, a discriminating portion for discriminating a person standing in front of the urinal to discharge a urine and a control portion so that a quantity of urine discharged from a specified person may be measured when said person discharging a urine is said specified person registered in said control portion in advance.

According to the above described construction, the specified person, such as patient, is equipped with a magnetic ID card on a part of a body thereof and said ID card is registered in the control portion such as CPU in advance. And, as soon as the patient stands in front of the urinal, it is discriminated by means of said discriminating portion, such as microwave discriminating device, provided in the urinal that the patient is the specified person and a closing valve of said quantity of urine-measuring portion provided in said drain outlet of the urinal is closed on the basis of the discriminating result to measure the quantity of urine discharged from the patient. Upon completing the appointed measurement, said closing valve is opened to discharge said urine into an exhaust passage. In addition, the measured result in the quantity measuring portion is operated together with ID data in said CPU to put data, such as a name of a person discharging the urine, a urine-discharging time, a quantity (cc) of urine for every urine-discharging time and summed up and a judgement of abnormality by a comparison with the suitable value, into a memory of the CPU. Said data put into said memory of the CPU are printed out or displayed on a display as occasion demands to be used as reference materials for a medical treatment of the patient and a pathological investigation.

And, in the case where patients and general persons without the magnetic ID card stand in front of the urinal, it is not discriminated that they are the specified person, so that the closing valve of the quantity measuring portion is not closed and thus the urine discharged from them is discharged into an exhaust passage without being measured, that is the urinal functions as a usual one.

### BRIEF DESCRIPTION OF THE DRAWINGS

One preferred embodiment of the present invention is shown in Figs. 1 to 4, in which
Fig. 1 is a diagram roughly showing a construction of an automatic quantity of urine-measuring apparatus according to the present invention;
Fig. 2 is an enlarged diagram showing a construction of a quantity of urine-measuring portion;
Fig. 3 is a block diagram showing a control relation in an automatic quantity of urine-measuring apparatus; and
Fig. 4 is a diagram showing one example of a recording format in a CPU.

### DETAILED DESCRIPTION OF THE PREFERRED EMBODIMENTS

The preferred embodiments of the present invention will be below described with reference to the drawings.

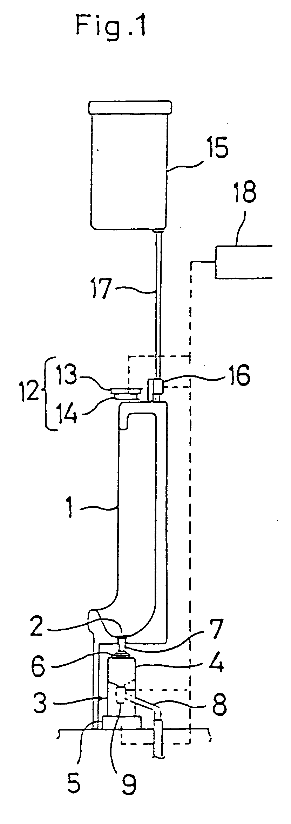

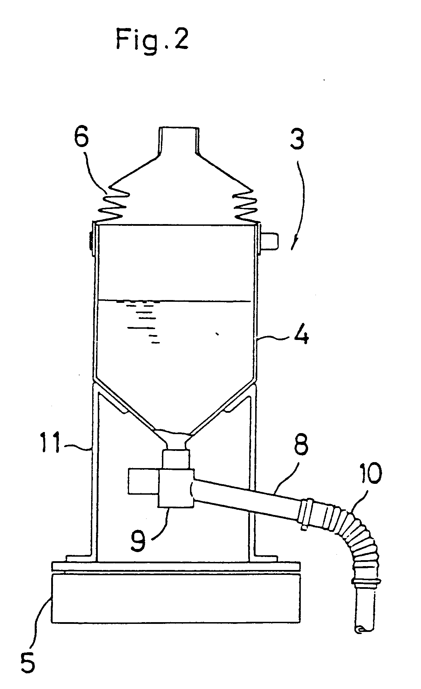

One preferred embodiment of the present invention is shown in Figs. 1 to 4. At first, Fig. 1 is a diagram roughly showing a construction of an automatic quantity of urine-measuring apparatus according to the present invention. Referring to Fig. 1, reference numeral 1 designates a urinal provided with a quantity measuring portion 3 disposed below a drain outlet 2 thereof. Said quantity measuring portion 3 comprises a measuring container 4 and an electronic balance 5 for placing said measuring container 4 thereon to measure it. And, an upper portion of the measuring container 4 is formed of a bellows portion 6 and said bellows portion 6 is communicated with a drain pipe 7 hanging down from a drain outlet 2. In addition, a bottom portion of the measuring container 4 is communicated with a drain pipe 8 and said drain pipe 8 is provided with a closing valve 9, such as electro-magnetic valve, and a flexible pipe 10 of which downstream side is connected with an exhaust passage (not shown). Furthermore, reference numeral 11 designates a support member for stably supporting the measuring container 4.

Reference numeral 12 designates a discriminating portion disposed in the vicinity of said urinal 1, for example above the urinal 1, and comprising a human body-detecting sensor 13, for example an infrared sensor, and a microwave discriminating device 14 for discriminating whether a person standing in front of the urinal 1 to discharge a urine is a specified person or not. Reference numeral 15 designates a tank connected with an upper portion of the urinal 1 through a supply pipe 17 provided with a closing valve 16, such as electro-magnetic valve, for storing water for washing the urinal.

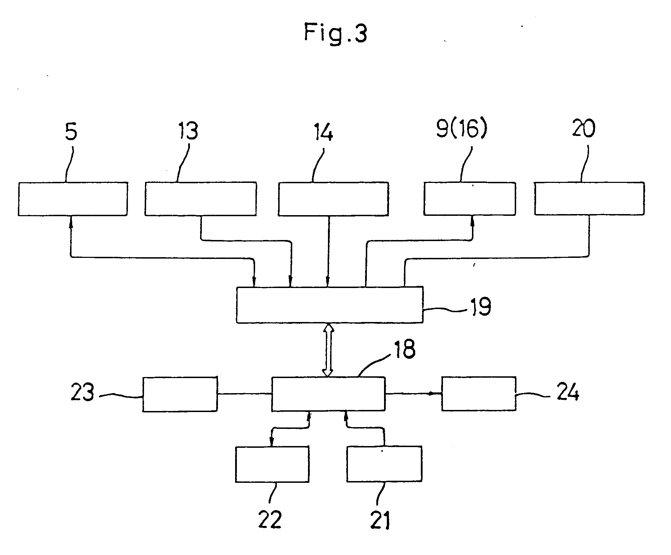

Fig. 3 roughly shows a control relation in an automatic quantity of urine-measuring apparatus having the above described construction. Referring to Fig. 3, reference numeral 18 designates a CPU as a control portion connected with not only said electronic balance 5, said human body-detecting sensor 13, said microwave discriminating device 14, said closing valve 9, said closing valve 16, a use of urinal-allowing display lamp 20 and the like through an interface 19 but also a keyboard 21 as an input device, a disk 22 for putting data thereinto, a printer 23 as an output device and a CRT 24 as a display. And, said CPU 18 conducts an operation of data on the basis of an input from the electronic balance 5 to control various kinds of sequence, that is not only to put the result of said operation of data into said disk 22, display it on said CRT 24 or putout it by means of said printer 23 but also to controlledly open and close the closing valves 9, 16 on the basis of a signal from the human body-detecting sensor 13 and the microwave discriminating device 14.

An operation of said automatic quantity of urine-measuring apparatus having the above described construction will be below described.

Provided that the specified person, such as the patient whose quantity of urine is to be measured, is equipped with a magnetic ID card (not shown) readable by microwaves on a part of a body thereof, for example a breast, and a name of the specified person is registered in the CPU 18 in advance together with classification codes (sex distinction, attachment, specified code number and the like), as soon as the patient equipped with said magnetic ID card stands in front of the urinal 1, said human body-detecting sensor 13 provided in the urinal 1 is operated not only to detect a start of a use of the urinal 1 but also to discriminate it by means of the microwave discriminating device 14 that the person standing in front of the urinal 1 is the specified one, whereby putting an appointed discriminating signal in the CPU 18. An appointed signal is put out from the CPU 18 on the basis of the above described input to immediately close the closing valve 9 of said quantity measuring portion 3, whereby collecting a urine discharged by the patient within the measuring container 4. Since a measured value by the electronic balance 5 when the measuring container 4 is empty is different from that when the urine is collected within the measuring container 4, the difference is measured as a weight of the urine and a measured output at this time is sent to the CPU 18.

And, as soon as the patient goes away from the front of the urinal 1, the completion of said use of the urinal 1 is detected to open the closing valve 9 and the closing valve 16 on the basis of the signal from the CPU 18, whereby discharging the urine within the measuring container 4 into said exhaust passage, and to open the closing valve 16, whereby supplying the urinal 1 with washings from said tank 15 and thus conducting an appointed washing.

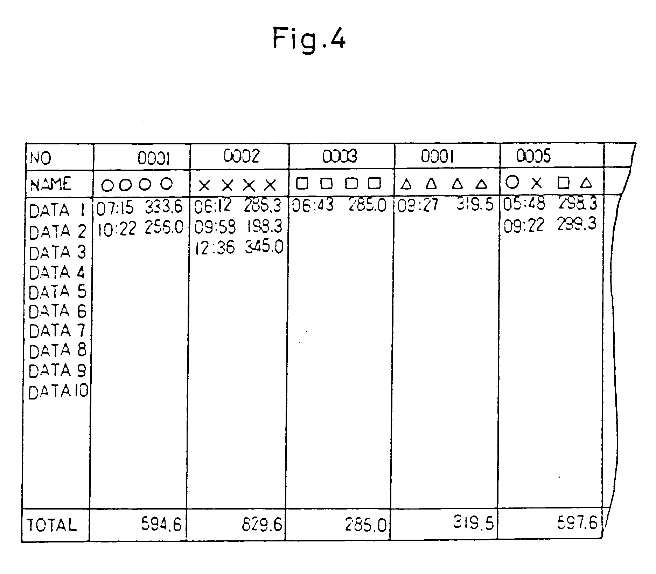

On the other hand, said measured output put out from the quantity measuring portion 3 is transformed into a volume value (cc) by the use of a specific gravity of urine in the CPU 18 to be recorded as data in a format as shown in Fig. 4 together with a urine-discharging time for every patient. As understood from Fig. 4, the measured result is operated in the CPU 18 together with the ID data for every registered specified person, such as patient, to be pigeonholed so that said urine-discharging time, a quantity of urine for every urine-discharging time and summed up and the like may be grasped at a glance. In addition, the judgement of abnormalities by the comparison with the suitable value and the like may be entered as occasion demands and these data are housed in the disk 22. The housed data are printed out by means of the printer 23 or displayed on the CRT 24 as occasion demands to be used as reference materials for a medical treatment of the patient and a pathological investigation.

And, in the case where patients and general persons without the magnetic ID card stand in front of the urinal 1, the human body-detecting sensor 13 provided in the urinal 1 is operated to detect the start of a use of the urinal 1 but the appointed discriminating signal is not put out from the microwave discriminating device 14, so that the closing valve 9 of the quantity of urine-measuring portion is not closed to discharge the discharged urine into the exhaust passage without being measured while as soon as the general persons go away from the front of the urinal 1, the completion of the use of the urinal 1 is detected to open the closing valve 16 on the basis of the signal from the CPU 18, whereby washing the urinal 1 with washings from the tank 15. In short, in this case, the urinal 1 functions as the general urinal.

The present invention is not limited by the above described preferred embodiment. For example, the measurement of urine may be conducted by a method using a liquid level indicator and a method using a liquid flow meter in place of the above described method using a balance. For example, in case of said method using a liquid level indicator, an ultrasonic method, a laser method and the like can be adopted for the detection of a liquid level. And, an impeller type flow meter and the like are used as said liquid flow meter to simplify the quantity measuring portion 3 in construction.

In addition, the specified person may be equipped with a microwave oscillator in place of the above described magnetic ID card as the disciminating means for discriminating whether the urine-discharging person is the specified person registered in advance or not.

And, the above described automatic quantity of urine-measuring apparatus may be provided in a plurality of urinal 1 to synthetically control these by means of the CPU 18.

The present invention has the above described construction, so that no wide space for keeping the containers, such as urinals, is required and the urine, has been subjected to the measurement, is let flow in the exhaust passage immediately in the same manner as the usual urine differently from the conventional urine-collecting method. Accordingly, a bad smell is not given out and no unpleasant feelings are given. In addition, the appointed measurement can be conducted without giving bitter feelings to the person in charge in the hospital and thus the labor-saving can be achieved.

Furthermore, the automatic quantity of urine-measuring apparatus according to the present invention has the superior advantages in that the function proper to the urinal is not spoiled at all even though it is equipped in the conventional urinal and it is sufficient to make a slight improvement when this apparatus is equipped.

## Claims

1. An apparatus for automatically measuring the quantity of urine, **characterized by** a urinal (1), a quantity measuring portion (3) provided in a drain outlet (2) of said urinal (1), a discriminating portion (12) for discriminating a person standing in front of the urinal (1) to discharge a urine and a control portion (18) so that a quantity of urine discharged from the person may be measured when said person discharging a urine is a specified person registered in said control portion (18) in advance.

2. An apparatus as set forth in claim 1, **characterized in that** said quantity measuring portion (3) comprises any one of a balance (5), a liquid level meter and a liquid flow meter.

3. An apparatus as set forth in claim 1 or 2, **characterized in that** a microwave discriminating device (14) as a part of said discriminating portion (12) is provided in the urinal (1) for determining whether the person is specified or not by the aid of a magnetic ID card with which the person is equipped.

## Patentansprüche

1. Vorrichtung zur automatischen Urinmengenmessung, **gekennzeichnet durch** ein Urinal (1), einen Mengenmeßabschnitt (3), der im Ablauf (2) des Urinals (1) vorhanden ist, einen Erkennungsabschnitt (12) zum Erkennen einer vor dem Urinal (1) zum Abgeben von Urin stehenden Person, und einen Steuerabschnitt (18), damit die von der Person abgegebene Urinmenge gemessen werden kann, wenn die den Urin abgebende Person eine spezielle Person ist, wie sie vorab im Steuerabschnitt (18) registriert wurde.

2. Vorrichtung nach Anspruch 1, **dadurch gekennzeichnet, daß** der Mengenmeßabschnitt (3) eine Waage (5), eine Flüssigkeitsniveau-Meßeinrichtung oder einen Flüssigkeits-Durchflußmesser beinhaltet.

3. Vorrichtung nach einem der Ansprüche 1 oder 2, **dadurch gekennzeichnet, daß** eine Mikrowellen-Erkennungsvorrichtung (14) als Teil des Erkennungsabschnitts (12) im Urinal (1) vorhanden ist, um zu bestimmen, ob die Person eine spezifizierte ist oder nicht, und zwar mit Hilfe einer magnetischen ID-Karte, mit der die Person versehen ist.

## Revendications

1. Un appareil pour mesurer automatiquement la quantité d'urine, caractérisé par un urinal (1), une partie de mesure de quantité (3) prévue dans une sortie d'évacuation (2) dudit urinal (1), une partie de détermination (12) permettant de distinguer une personne debout devant l'urinal (1) pour uriner de l'urine et une partie de contrôle (18) de sorte que l'on puisse mesurer la quantité d'urine urinée par cette personne lorsque ladite personne urinant de l'urine est une personne spécifiée, enregistrée à l'avance dans ladite partie de contrôle (18).

2. Un appareil selon la revendication 1, caractérisé en ce que ladite partie de mesure de quantité (3) comporte l'un quelconque des éléments parmi un appareil de pesée (5), un appareil de mesure de niveau (limnimètre) et un appareil de mesure de débit de liquide (débitmètre).

3. Un appareil selon la revendication 1 ou 2, caractérisé en ce qu'un dispositif de détermination à microondes (14) constituant une portion de ladite partie de détermination (12), est prévu dans l'urinal (1) pour déterminer, à l'aide d'une carte magnétique d'identification dont la personne est équipée, si cette personne est ou non la personne spécifiée.
